Europäisches Patentamt

European Patent Office

Office européen des brevets

(19)

(11) EP 0 514 691 B1

(12) EUROPEAN PATENT SPECIFICATION

(45) Date of publication and mention
of the grant of the patent:
**03.01.1996 Bulletin 1996/01**

(51) Int Cl.6: **C08J 5/18**, A61L 15/32,
A61L 27/00, A61L 25/00,
C08L 89/06

(21) Application number: 92107249.2

(22) Date of filing: **29.04.1992**

(54) **Non-porous collagen sheet for therapeutic use, and the method and apparatus for preparing it**

Nichtporöse Kollagenfolie zur therapeutischen Verwendung, und Verfahren und Vorrichtung zu ihrer Herstellung

Feuille non poreuse de collagène à usage thérapeutique, et procédé et appareil pour sa préperation

(84) Designated Contracting States:
**AT BE CH DE DK ES FR GB GR IT LI LU MC NL PT SE**

(30) Priority: **23.05.1991 IT MI911423**

(43) Date of publication of application:
**25.11.1992 Bulletin 1992/48**

(73) Proprietor: **EURORESEARCH S.r.L.**
**I-20145 Milano (IT)**

(72) Inventors:
• **Furlan, Diego**
**I-20090 Segrate, (Milan) (IT)**
• **Bonfanti, Giovanni**
**I-04023 Formia Santa Croce, (Latina) (IT)**
• **Scappaticci, Giuseppe**
**I-03043 Cassino, (Frosinone) (IT)**

(74) Representative: **Gervasi, Gemma, Dr. et al**
**I-20122 Milano (IT)**

(56) References cited:
**EP-A- 0 376 931**          **DE-A- 1 945 072**
**FR-A- 2 160 462**          **US-A- 4 948 540**

Remarks:
The file contains technical information submitted after the application was filed and not included in this specification

## Description

Collagen is a scleroprotein widespread in nature. It represents about one third of the total proteins of the human body.

Medical practice has recently seen the introduction of the use of collagen as a stimulating agent in the cicatrization process involving an interaction effect with various growth factors, because of its capturing action on fibronectin, a glycoprotein which promotes cell attachment and the migration and replication of the resultant cells (see "Il collageno nella cicatrizzazione" by B. Palmieri, publ. Artestampa, January 1990, pp. 40-42) and other actions which are still not totally clear. The known collagen product, using a particular non-denaturing process, is prepared in stable form by a process of extraction from animal organs rich in this scleroprotein, purification and subsequent lyophilization. The final product is in the form of mats of greater or lesser thickness, characterised by high absorbent power (exudates and liquids in general) because of its structure in the form of fibres which are spaced apart and branched in such a manner as to make a large specific surface available for absorption (up to 50 times its weight). The hydrophilic nature of collagen also greatly favours this absorbent power.

In addition to the aforesaid function, the role of collagen in cicatrization is characterised by collagen/platelet interaction and the formation of a bond between the collagen, the fibronectin and the growth factors, molecules which are known to be implicated in regulating the cicatrization process (see pages 45-46 of the aforesaid text).

There are however cases in which the absorbent formation of the collagen sponge and its hydrophilic nature lead to an excessive loss of physiological liquids. It is well known that an evaporation process normally occurs through the undamaged skin, and this increases considerably in the case of skin lesion, resulting in dehydration of the underlying layers. The phenomenon is accentuated for example in burn cases, when large skin portions are damaged traumatically. In this case the absorbent effect of lyophilized collagen further increases the process of evaporation, with consequent damage to the underlying structure.

Another kind of known collagen-based medicament useful for wound healing is represented by the collagen membranous material disclosed by EP-A-0376931, which is prepared by disrupting and centrifuging a collagen gel matrix so to precipitate collagen, homogenizing the precipitated material in the form of a paste, casting and drying the paste.

The present invention provides a product which while maintaining the rapid cicatrization characteristics of collagen, at the same time prevents excessive evaporation, allows constant inspection of the bed of the wound without having to be removed (transparency), is simple and practical to use, adheres satisfactorily to the injured surface, does not require frequent replacement, can transpire to allow oxygenation of the bed of the wound while preventing its contamination by bacteria, is absorbable but not soluble in the biological liquids with which it comes into contact, unless by specific enzymatic action, and is structurally homogeneous.

Another important characteristic of the collagen according to the invention is that of being suitable as interposition material for preventing accretions in the internal surgery operations.

To obtain a product with these characteristics, type I collagen was used as defined in Table 1 on page 3 of the aforestated text, that is having molecular structure $[\alpha1(I)_2]\alpha2(I)$, this having the characteristic of being insoluble in the various types of biological liquids. Type I collagen present in the skin represents about 80% of the total located in the deep dermis, 90-95% in the tendons and 100% in the bones. Type I collagen is therefore the most biologically similar to that present in the human skin.

Because of its insolubility, in order to obtain a product of homogeneous structure, use was made of the known method of dispersing fibrous collagen in a dilute acetic acid solution of about pH 2.5 and maintaining agitation until a good dispersion of the collagen fibres in the liquid is obtained. At this pH value the fibres swell to form a gel. The gel obtained, still comprising fibre fractions which have not completely gelled and possibly corpuscles of extraneous substances, is further diluted with an acetic acid solution of pH 2.5-3.5 until a sufficiently fluid mass is obtained, which is then filtered.

The filtering, which is done under vacuum, uses a special filter, indicative (but not limitative) characteristics of which are given hereinafter, and allows practically total elimination of the inevitable air bubbles which form during gelling and are difficult to eliminate given the viscosity of collagen gel.

By the effect of the vacuum, which has to be of the order of 3999 Pa residual pressure, these bubbles increase their volume, the passage through the mesh then breaks down and eliminates them. It has been found experimentally that the best filtration conditions to achieve the described phenomenon are a gel temperature of 10-30°C, preferably 25-28°C, and a residual vacuum of 2666-7998 Pa (20-60 mmHg), preferably about 3999 Pa (30 mmHg).

These data are indicative and have been found experimentally to be the most effective, although not representing a limitation on the operating conditions of this process.

The filtered gel is collected in a closed vessel maintained under vacuum and constructed in such a manner that the filtered gel runs along vessel partition walls located below the filter mesh and structured to produce a continuous liquid film which does not allow further air absorption after filtration, following inclusion of air bubbles.

The filtered gel is further maintained under vacuum at 2666-3332.5 Pa (20-25 mmHg) for a further hour to allow total elimination of any air bubbles which may still be present in the gel.

FILTER APPARATUS

The filter required for filtering the collagen gel, which besides eliminating the solid particles, which are retained on the mesh, also eliminates the air bubbles contained in it, consists of an upper cylindrical stainless steel shell provided with a scraping stirrer to keep the collagen gel mixed and to remove solid particles from the mesh so that they do not clog it. The bottom of the cylindrical shell houses a stainless steel mesh with a mesh size of less than 0.1 mm (Taurail meshes have been found to be particularly effective).

The lower part (below the mesh) consists of a cylindrical shell in which vacuum can be generated by a suitable pump. The air bubbles contained in the gel which filters through the mesh increase considerably in volume because of the vacuum.

At about 3 mm below the filter mesh there is a device consisting of a series of stainless steel plates which are vertically or raking placed and parallel between them. The filtered gel descends along these plates in the form of a continuous liquid film and runs by gravity towards the bottom of the vessel.

Those air bubbles which do not break down by the effect of the reduced pressure remain mainly in the upper part of the device whereas the gel, now free or almost free of air, runs to the bottom of the vessel. Any very small bubbles still present in the filtered gel decrease considerably in volume when returned to atmospheric pressure, so that they become practically absent.

In this respect, during filtration because of the difference between the pressure of the gel environment before filtration and the residual pressure below the mesh (about 3999 Pa), the bubble volume increases more than 25 times. Likewise, on passing from vacuum to the environmental pressure the bubble volume decreases 25 times. Hence the air bubbles of diameter less than 0.100 mm (advisable mesh passage size) have a diameter of less than 0.034 mm when returned to atmospheric pressure, ie are practically invisible. During drying, these residual bubbles are eliminated without leaving appreciable craters in the structure of the obtained sheet.

This means that extremely uniform thicknesses can be obtained over the entire sheet surface, so avoiding any porosity which could represent a point of preferential attack by enzymatic action, which would annul the protective effect against invasion by micro-organisms.

DRYING

The filtered gel obtained as described, free from extraneous particles and air bubbles and perfectly clear and transparent, can then be used for preparing films of desired thickness and diameter. For this, after analysis to exactly determine the concentration of the filtered gel, exactly measured quantities for obtaining films with the desired collagen thickness must be metered into suitable containers. This metering is generally effected by a suitable peristaltic pump which prevents incorporating air into the gel while at the same time preventing heating or friction which could damage the structure of the collagen protein. The containers are of tray shape and are formed of antiadherent material.

The described trays loaded with the gel in a controlled environment (relative humidity 60-80%, temperature 20-22°C, environment class 10,000 or less) are placed in a suitable controlled drying oven where they are left to stand for at least two hours to obtain perfect gel thickness uniformity. The oven is purged with a nitrogen stream for about 30 minutes to totally eliminate air and remove oxygen, in order to ensure constant operating conditions and prevent possible oxidation.

This operation has also been shown to practically totally block the growth of micro-organism colonies, which sometimes occurs if the procedure is carried out with air present in the environment. Drying is effected in a nitrogen stream under closed cycle.

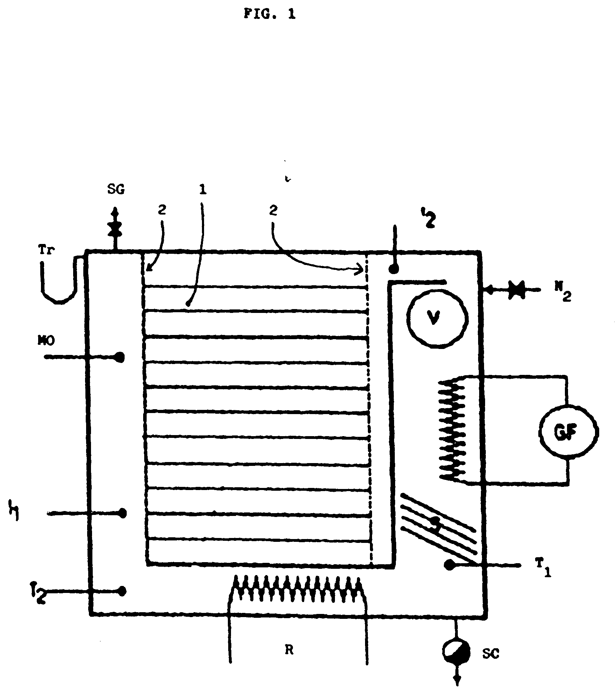

The drying, being the critical stage for obtaining films with the desired characteristics, is conducted under particular conditions in an appropriate oven shown schematically in Figure 1.

In this, the reference numeral 1 indicates the drying trays resting on perforated side walls, V indicates the fan for circulating nitrogen through the apparatus, $N_2$ indicates the nitrogen feed valve, GF indicates the refrigeration unit with coil, S represents a parallel plate device for separating condensate droplets, $T_1$ indicates a first thermometer, SC indicates the condensed water discharge, R indicates the heating device, $T_2$ indicates a second thermometer, $I_1$ indicates a first hygrometer, MO indicates an oxygen meter (analyzer), Sg indicates the gas discharge, Tr indicates an overpressure trap and $I_2$ indicates a second hygrometer.

The oven is arranged in this manner to satisfy the following requirements:

1) the facility for eliminating air by purging with nitrogen to a residual oxygen content of less than 2%;

2) the facility for varying the nitrogen cooling and heating temperature to a maximum of 30°C, to control the relative humidity in the drying chamber and the water evaporation rate;

3) the facility for regulating the rate of nitrogen circulation through the chamber so as not to create high flow points and hence maintain a uniform drying rate over the entire surface and prevent the formation of creases which, besides being undesirable from the appearance aspect, are an indication of different collagen concentrations and poor homogeneity of drying (localized drying).

The $H_2O$ content of the product must not be higher than 20% by weight. It is preferable to achieve a higher level of drying (down to 2% or 3% of $H_2O$), in particular to ensure proper elimination of the acetic acid present in the initial gel. The dried product obtained easily reabsorbs moisture from the environment, while being maintained within the maximum limit of 20%.

EXAMPLE

The conditions found experimentally to be most appropriate for conducting a drying cycle are given below by way of non-limiting example.

1st stage:

Nitrogen purging until the oxygen content is less than 1%, standing for two hours to come to equilibrium, loaded gel level 10 mm, gel collagen concentration 0.5%.

2nd stage:

Starting of nitrogen circulation by fan.
Nitrogen temperature after cooling -5°C ($T_1$).
Nitrogen temperature after heating 26-28°C ($T_2$).
Time about 12 hours.
Relative humidity entry to drying region (point $I_1$) 12-14%.
Relative humidity exit of drying region (point $I_2$) 70-80%.

3rd stage:

Nitrogen temperature after cooling -15°C ($T_1$).
Nitrogen temperature after heating 26-28°C ($T_2$).
Time about 12 hours.
Relative humidity entry to drying region (point $I_1$) 6-7%.
Relative humidity exit of drying region (point $I_2$) 45-50%.

4th stage:

Final drying

Nitrogen temperature after cooling -40°C ($T_1$).
Nitrogen temperature after heating 26-28°C ($T_2$).
Time about 12 hours.

5th stage:

Product discharge, preparation of a new load. Complete removal of water from the cooling coil and purging the oven by nitrogen circulation at 70-80°C for two hours, cooling to 20°C and loading new product.
The nitrogen flow rate through the drier is adjusted on the basis of the required degree of drying.
A semi-transparent film with a thickness of about 200 micron is obtained. The thickness can vary in general between 0.02 and 2 mm. This represents a non-specific item for the purposes of the therapeutic application as it determines only the product absorption time but not its specific characteristics. The degree of drying can also vary as stated.
The characteristics of the film obtained are:

- maintaining of the "native" structure of collagen fibre (the classical triple spiral structure of collagen has been demonstrated by the electron microscope)

- absence of degradation products such as monomers or dimers of collagen not organized into fibrils, or gelatin, an indication of potential allergenicity

- high protein nitrogen content (exceeding 90%)

- high hydroxyproline content (exceeding 12%)

- low absorbent power (about 10-15 times its weight against 50 times for the lyophilized product of the known art)

- high resistance to enzymatic attack

- good product transparency

- excellent plasticity after immersion in physiological solution.

The product obtained in this manner is sterilized by irradiation with gamma rays and used in the treatment of burns and generally all cases of skin removal or damage.
The result is excellent both in terms of tolerance (no case of allergenicity or hypersensitivity to the medicament has been recorded, the native characteristic of the product remaining unaltered during the process) and in terms of pain attenuation. The cicatrization time is very rapid and product absorption considerably longer compared with equivalent treatment using lyophilized collagen (sponge) and consequently there is lesser need to replace it. Exudate loss is very low, and much lower than that when using lyophilized collagen.
The transparency of the product means that the progress of the injury can be viewed without the need to remove the collagen sheet (generally a painful procedure).
The product can be presented in the form of sheets of different dimensions (square, rectangular, round, elliptical or others) supported or not supported by adhesives (such as plasters) or by sheets of inert substances such as nylon, polyurethane, polyethylene etc., or associated during the drying process, or subsequently, with pharmacologically active substances.

## Claims

1. A sheet of type I collagen gel, having molecular structure $[\alpha 1(I)]_2 \alpha 2(I)$, suitable for the therapeutic cicatrizing treatment of wounds and burns, said sheet being free from native collagen degradation products, having an $H_2O$ content not exceeding 20% by weight, a uniform thickness, comprised between 0.02 and 2 mm, said sheet being characterized in that it is of transparent structure, it has an homogeneous structure, it has the classical triple-helical structure of native collagen, it comprises gas bubbles with a diameter of less than 0.034 mm under atmospheric pressure, it has a capacity for absorbing aqueous biological liquids limited to a maximum of 15 times its weight and a high resistance to enzymatic attack.

2. A method of preparing a sheet of type I collagen as claimed in claim 1 from aqueous diluted collagen gel of pH 2.5-3.5, comprising filtering the gel through a filter surface with a passage size of less than 0.1 mm, the lower part of filtering apparatus being under a vacuum of 2666-7998 Pa and provided in the region immediately below the filter mesh with a device consisting of a series of plates vertically or raking placed and parallel between them, then drying the liquid gel contained in trays by purging with nitrogen to a residual oxygen content of less than 2%, at a temperature of 20-22°C and with a relative humidity of 60-80%.

3. A device suitable for filtering under vacuum the collagen gel in accordance with claim 2, comprising an upper shell provided with a scraping stirrer, a metal mesh with a mesh size of less than 0.1 mm placed at the bottom of the upper shell, a lower shell connected with a vacuum pump, and provided in the region immediately below the filter mesh with a pack of plates vertically or raking placed and parallel between them for the purpose of conveying the filtrate constitued by the said collagen gel as a continuous liquid film.

4. Use of the sheet of type I collagen as claimed in claim 1, for the preparation of a medicament useful for the treatment of wounds and burns and as interposition material for preventing accretions in the internal surgery operations.

## Patentansprüche

1. Folie aus Typ I-Kollagengel, das die Molekularstruktur $[\alpha 1(I)]_2 \alpha 2(I)$ hat und für die therapeutische Narbenbildungsbehandlung geeignet ist, wobei die Folie von Abbauprodukten natürlichen Kollagens frei ist, einen Wassergehalt hat, der 20 Gew.-% nicht übersteigt; eine gleichmäßige Dicke zwischen 0,02 und 2 mm hat,
dadurch gekennzeichnet, daß
die Folie eine transparente Struktur aufweist, eine homogene Struktur hat, daß sie die klassische Dreifachhelix-Struktur von natürlichem Kollagen hat, bei atmosphärischem Druck Gasblasen mit einem Durchmesser von weniger als 0,034 mm enthält, ihre Kapazität zur Absorption wäßriger biologischer Flüssigkeit auf ein Maximum des 15-fachen ihres Gewichts limitiert ist, und sie eine hohe Beständigkeit gegenüber einem enzymatischen Angriff hat.

2. Verfahren zur Herstellung einer Folie aus Typ I-Kollagengel nach Anspruch 1 aus wäßrigem verdünntem Kollagengel mit einem pH von 2,5 bis 3,5, umfassend

   - Filtrieren des Gels durch eine Filteroberfläche mit einer Durchgangsgröße von weniger als 0,1 mm, wobei der untere Teil der Filtrierapparatur unter einem Vakuum von 2666 bis 7998 Pa steht und in dem Bereich unmittelbar unter dem Filtersieb bereitgestellt ist, mit einer Vorrichtung, die aus Reihen von Platten besteht, welche senkrecht oder schräg und zueinander parallel angeordnet sind;

   - Trocknen des flüssigen Gels, das in Schalen enthalten ist, durch Spülen mit Stickstoff bis zu einem Restsauerstoffgehalt von weniger als 2%, bei einer Temperatur von 20 bis 22°C und einer relativen Feuchtigkeit von 60 bis 80%.

3. Vorrichtung, die zum Filtrieren des Kollagengels nach Anspruch 2 geeignet ist, und die aus einem oberen Mantel, der mit einem Shabrührer ausgestattet ist; einem Metallsieb mit einer Maschengröße von weniger als 0,1 mm, das am Boden des oberen Mantels angeordnet ist; einem unteren Mantel, der mit einer Vakuumpumpe verbunden ist, besteht, und die im Bereich unmittelbar unter dem Filtersieb mit einer Reihe von Platten, die vertikal oder schräg und zueinander parallel angeordnet sind, zum Zwecke eines Beförderns des Filtrats, das aus dem Kollagengel besteht, als kontinuierliche flüssige Folie, versehen ist.

4. Verwendung der Folie aus Typ I-Kollagengel nach Anspruch 1 zur Herstellung eines Medikaments, das zur Behandlung von Wunden und Verbrennungen sowie als Interpositionsmaterial zur Verhinderung von Verwachsungen bei inneren Operationen verwendbar ist.

**Revendications**

1. Feuille de gel collagène du genre I ayant une structure moléculaire $[\alpha 1(I)]_2 \alpha 2(I)$, apte au traitement thérapeutique cicatrisant de blessures et brûlures, la susdite feuille étant sans produits de dégradation du collagène naturel, ayant un contenu de $H_2O$ qui n'est pas supérieur à 20% en poids et une épaisseur uniforme de 0,02-2 mm, la susdite feuille étant characterisée en ce qu'elle a une structure transparente et homogène, la structure typique à triple-hélice de collagène naturel, en ce qu'elle comprend des bulles de gaz ayant un diamètre de moins de 0,034 mm à pression atmosphérique, et en ce qu'elle est capable d'absorber des liquides biologiques aqueux pour un maximum de 15 fois son poids et est très resistante à l'attaque enzymatique.

2. Méthode pour préparer une feuille de collagène du genre I selon la revendication 1 à partir de gel collagène aqueux dilué avec un pH 2,5-3,5, comprenant la filtration du gel à travers une surface filtrante avec un passage inférieur à 0,1 mm, la partie inférieure de l'appareil filtrant étant sous un vide de 2666-7998 Pa et étant pourvue dans la région directement au-dessous du filet de filtration d'un appareil composé par une série de plaques arrangées verticalement ou inclinées et parallèles entre elles, la susdite méthode comprenant ensuite le séchage du gel liquide contenu dans des plateaux en le purgeant avec azote jusqu'à obtenir un contenu en oxygène inférieur à 2% à une température de 20-22°C et avec une humidité relative de 60-80%.

3. Appareil apte à la filtration sous vide du gel collagène selon la revendication 2, comprenant une coque supérieure avec un agitateur raclant, un filet métallique avec une maille inférieure à 0,1 mm placé au fond de la coque supérieure, une coque inférieure liée avec une pompe à vide et pourvu dans la région directement au-dessous du filet de filtration d'une pile de plaques arrangées verticalement ou inclinées et parallèles entre elles pour acheminer le filtrat constitué par le susdit gel collagène comme une pellicule liquide continue.

4. Emploi de la feuille de collagène du genre I selon la revendication 1 pour la préparation d'un médicament utilisé dans le traitement de blessures et brûlures et comme matériel d'interposition pour éviter des excroissances dans les opérations de chirurgie interne.

FIG. 1